# EUROPÄISCHE PATENTANMELDUNG

(11) **EP 3 935 945 A1**
(43) Veröffentlichungstag der Anmeldung: **12.01.2022**
(21) Anmeldenummer: 20184435.4
(22) Anmeldetag: 07.07.2020
(51) Int. Cl.: A01N 59/04, C12F 3/02

(54) **ZUSAMMENSETZUNG ZUR HERSTELLUNG VON CO2, VERWENDUNG EINER ZUSAMMENSETZUNG ZUR HERSTELLUNG VON CO2 UND VERFAHREN ZUR HERSTELLUNG VON CO2**

(71) Anmelder: Biogents Aktiengesellschaft, 93055 Regensburg (DE)
(72) Erfinder: GEIER, Martin, 92237 Sulzbach-Rosenberg (DE); ROSE, Andreas, 93053 Regensburg (DE); GORDON, Scott W., 93057 Regensburg (DE); EPPLE, Maximilian, 93055 Regensburg (DE)
(74) Vertreter: Benninger, Johannes

(57) **Zusammenfassung**

Die Erfindung bezieht sich auf eine Zusammensetzung zur Herstellung von CO₂, die Verwendung einer Zusammensetzung zur Herstellung von CO₂ für eine Insektenfalle, insbesondere für eine Insektenfalle zum Anlocken blutsaugender Insekten und Arthropoden, und ein Verfahren zur Herstellung von CO₂. Die Zusammensetzung um fasst eine Komponente a), eine Komponente b) und eine Komponente c). Die Komponente a) umfasst mindestens einen ersten Hefestamm, der eine geringe Toleranz von weniger als 100g Alkohol pro Liter aufweist. Die Komponente b) umfasst mindestens einen zweiten Hefestamm, der eine hohe Toleranz von mehr als 100g Alkohol pro Liter aufweist. Die Komponente c) umfasst mindestens eine Nährstoffquelle für den mindestens einen ersten Hefestamm und/oder für den mindestens einen zweiten Hefestamm.

## Beschreibung

Die vorliegende Erfindung betrifft eine Zusammensetzung zur Herstellung von CO₂, die Verwendung einer Zusammensetzung zur Herstellung von CO₂ und ein Verfahren zur Herstellung von CO₂ gemäß den Merkmalen des der unabhängigen Ansprüche.

Blutsaugende Insekten richten sich häufig nach olfaktorischen und/oder optischen Reizen, um Menschen oder Tiere aufzuspüren. Problematisch ist insbesondere, dass blutsaugende Insekten häufig Krankheiten übertragen, weshalb versucht wird, diese Insekten von menschlichen Behausungen und/oder Ansammlungen fern zu halten. Dabei kann entweder auf die Abwehr gesetzt werden, indem beispielsweise chemische Insektenabwehrmittel eingesetzt werden. Alternativ werden unterschiedliche Insektenfallen eingesetzt, um blutsaugende Arthropoden einzufangen. Solche Insektenfallen arbeiten häufig mit olfaktorischen und/oder optischen Lockstoffen.

Es ist beispielsweise bekannt, dass eine Kombination von Kohlendioxid und Milchsäure in der Lage ist, eine Reihe blutsaugender Insektenarten anzulocken. Beispielsweise beschreibt die US-Patentschrift mit der Veröffentlichungsnummer US 4 907 366 A die Verwendung einer Zusammensetzung aus Milchsäure, Kohlenstoffdioxid, Wasser in Verbindung mit Wärme, um Stechmücken anzulocken.

Um Kohlenstoffdioxid für die Verwendung in Insektenfallen bereitzustellen, ist beispielsweise die Verwendung von industriellen Gasflaschen bekannt. Dies ist jedoch relativ teuer und technisch sehr aufwendig. Aus diesem Grund ist auch die Verwendung von Mikroorganismen wie Hefe bekannt, die aus einem entsprechenden Substrat durch Verstoffwechslung, insbesondere Gährung und/oder Atmung, Kohlenstoffdioxid herstellen.

Die Offenlegungsschrift WO 99/26471 A1 beschreibt eine Insektenfalle, bei welcher Bäckerhefe in einer Nährlösung verwendet wird, um eine kontinuierliche Kohlenstoffdioxid Abgabe zu erzeugen.

Die Aufgabe der Erfindung besteht darin, besonders einfach, kostengünstig und schnell Kohlenstoffdioxid zu erzeugen, insbesondere für die Verwendung in oder mit Insektenfallen.

Die obige Aufgabe wird durch eine Zusammensetzung zur Herstellung von CO₂, die Verwendung dieser Zusammensetzung und ein Verfahren zur Herstellung von CO₂ gelöst, die die Merkmale in den unabhängigen Patentansprüchen umfassen. Weitere vorteilhafte Ausgestaltungen werden durch die Unteransprüche beschrieben.

Die erfindungsgemäße Zusammensetzung umfasst
- eine Komponente a) mit mindestens einem ersten Hefestamm, welcher mindestens eine erste Hefestamm eine geringe Toleranz von weniger als 100g Alkohol pro Liter aufweist,
- eine Komponente b) mit mindestens einem zweiten Hefestamm, welcher mindestens eine zweite Hefestamm eine hohe Toleranz von mehr als 100g Alkohol pro Liter aufweist,
- eine Komponente c) umfassend mindestens eine Nährstoffquelle für den mindestens einen ersten Hefestamm und/oder für den mindestens einen zweiten Hefestamm.

Insbesondere ist der in der Komponente a) enthaltene mindestens eine erste Hefestamm für die Produktion von Kohlenstoffdioxid optimiert, wobei die Produktion von Kohlenstoffdioxid durch den ersten Hefestamm vorzugsweise besonders gleichmäßig erfolgt.

Beispielsweise wird die Komponente a) durch Bäckerhefe gebildet, die insbesondere darauf optimiert ist, unter Luftabschluss und in kurzer Zeit viel Kohlenstoffdioxid zu erzeugen. Diese kann in der Zusammensetzung in feuchter Form, beispielsweise als gepresste Frischhefe, als Flüssighefe oder als aktive Trockenhefe enthalten sein. Neben den Hefezellen enthält kommerziell erhältliche Bäckerhefe immer auch Mineralien wie Kalium, Phosphor, Magnesium, Calcium etc., welche insbesondere aus dem Nährmedium stammen, in dem die Hefezellen angezüchtet wurden. Insbesondere startet die CO₂ Produktion bei Bäckerhefe im Wesentlichen sofort nach dem Zumischen der Bäckerhefe zu einer Kohlenhydratlösung und steigt bei Kohlenhydratüberschuss im Wesentlichen kontinuierlich und gleichmäßig an.

Weiterhin ist der in der Komponente b) enthaltene mindestens eine zweite Hefestamm für die Produktion von Alkohol optimiert. Vorzugsweise umfasst die Komponente b) mindestens eine sogenannte Brennhefe, eine Bierhefe und/oder eine Weinhefe, die insbesondere eine Alkoholtoleranz zwischen 110 Gramm pro Liter und 120 Gramm pro Liter aufweist.

Vorzugsweise kann die Komponente b) durch eine so bezeichnete Turbohefe gebildet werden. Derartige sog. Turboheben werden speziell für den Brauereibetrieb vertrieben. Die hier verwendete Bezeichnung "Turbohefe" stellt dabei einen Sammelbegriff dar und bezeichnet eine Mischung aus einer Hefe und einer Hefenahrung. Die Bezeichnung "Turbohefe" bezeichnet daher nicht eine gewisse Hefe, sondern ein Gemisch aus einer Hefe, beispielsweise einer Reinzuchthefe, und komplexer Hefenahrung. Beispielsweise besteht eine solche Turbohefe zu 58% aus einer Brennhefe, 20% Carbamid, 16% Phosphaten, 3% Sulfaten, 2% Karbonaten, sowie 1% Vitaminen und Spurenelementen.

Die sogenannten Turbohefen enthalten mindestens einen Hefestamm, der mindestens 14% Alkohol toleriert, teilweise tolerieren die Turbohefen bis zu 20% Alkohol oder sogar mehr. Turbohefen sind darauf optimiert, dass im Endeffekt viel Alkohol produziert wird. Allerdings startet bei Verwendung von Turbohefe allein die Produktion von Kohlenstoffdioxid erst verzögert.

Weiterhin ist es vorteilhaft, wenn der erste Hefestamm und/oder der zweite Hefestamm osmotolerant sind und relativ hohe Anfangskonzentrationen an Zucker vertragen. Dadurch kann der gesamte als Kohlenhydratquelle dienende Zucker direkt auf einmal zugegeben werden und die Hefen müssen nicht mehrfach mit Kohlenhydrat nachgefüttert werden, um die entsprechende CO₂- Produktion über einen gewünschten Zeitraum beizubehalten.

Eine weitere bevorzugte Ausführungsform sieht vor, dass der erste Hefestamm und/oder der zweite Hefestamm nicht nur einfache Zucker wie Fruktose und Glukose verstoffwechseln können, sondern insbesondere auch gewöhnlichen, billigen Haushaltszucker mit der gleichen Schnelligkeit verstoffwechseln können. Eine weitere alternative Ausführungsform sieht vor, dass der erste Hefestamm und/oder der zweite Hefestamm auch andere Kohlenhydratquellen wie Mehl o.ä. schnell und effektiv verstoffwechseln können, beispielsweise Malzmehl, Getreidemehl, Maismehl oder Mehl aus anderen geeigneten Stärkequellen wie Tapioka, Yams, Maniok o.ä.

Gemäß einer bevorzugten Ausführungsform wird die Komponente c) durch eine Turbohefe gebildet. Wie oben beschrieben, umfassen die kommerziell erhältlichen Turbohefen neben einem Alkohol toleranten Hefestamm zusätzlich mindestens eine Nährstoffquelle für diesen Hefestamm. Eine Ausführungsform der Zusammensetzung kann somit vorsehen, dass die Zusammensetzung aus einem ersten Teil Bäckerhefe und einem zweiten Teil Turbohefe gebildet wird. Dabei können beispielsweise gleiche Teile an Bäckerhefe und Turbohefe eingesetzt werden. In Abhängigkeit von der gewünschten Kohlendioxidmenge und dem gewünschten Herstellungsprofil über die Zeit kann die Zusammensetzung vorteilhaft mehr Bäckerhefe oder aber mehr Turbohefe enthalten.

Gemäß einer bevorzugten Ausführungsform wird die Komponente c) durch einen Hefeextrakt gebildet. Unter Hefeextrakt ist ein Konzentrat der löslichen Inhaltsstoffe von Hefezellen zu verstehen. Hefeextrakt enthält einen hohen Anteil an Proteinen, Aminosäuren, Nukleotiden und Vitaminen der B-Gruppe. Hefeextrakt wird insbesondere aus überwiegend eiweißreichen, speziell gezüchteten Heferassen (Reinzuchthefe) hergestellt. Diese Hefen werden aufgeschlossen, wodurch der Zellsaft freigesetzt wird. Die hefeeigenen Proteasen und Hydrolasen hydrolysieren dann die Zellinhalte, wodurch Proteine in Peptide und Aminosäuren, DNA und RNA zu Nukleotiden aufgespalten werden. Um einen höheren Nukleotidgehalt zu erzielen, können die hefeeigenen Enzyme durch Zugabe von Nucleasen ergänzt werden. Das anfänglich flüssige Auto- bzw. Hydrolysat wird im weiteren Verlauf von den unlöslichen Zellbestandteilen befreit, filtriert, aufkonzentriert und gegebenenfalls sprühgetrocknet, so dass ein Trockensubstanzgehalt von 70-80 % bei pastösem Hefeextrakt, 95-97 % bei Pulver erreicht wird.

Eine Ausführungsform sieht vor, dass die Komponenten a : b : c jeweils in gefriergetrockneter Form vorliegen, beispielsweise umfasst die Zusammensetzung somit kommerziell erhältliche gefriergetrocknete Bäckerhefe, kommerziell erhältliche gefriergetrocknete Turbohefe und kommerziell erhältliche gefriergetrockneten Hefeextrakt.

Eine Ausführungsform sieht vor, dass die Komponenten a : b : c in einem Mengenverhältnis oder Gewichtsverhältnis von 1-10: 1-10: 1-10 in der Zusammensetzung vorhanden sind. Insbesondere ist ein Mengenverhältnis oder Gewichtsverhältnis 2: 1: 1 oder 1: 2: 1 oder 1: 1: 2 bevorzugt. Die angegebenen Mengenverhältnisse gelten insbesondere, wenn alle drei Komponenten vorzugsweise in gefriergetrockneter Form eingesetzt werden. Entsprechende Mengenverhältnisse gelten bei Verwendung der Komponenten in Frischform mit Feuchtigkeitsanteil oder bei Verwendung mindestens einer Komponente in gefriergetrockneter Form und einer Komponente in Frischform mit Feuchtigkeitsanteil. Gegebenenfalls müssen die Mengenverhältnisse angepasst werden, um dies entsprechend zu berücksichtigen.

Die vorbeschriebene Zusammensetzung kann immer vorteilhaft eingesetzt werden, wenn über einen längeren Zeitraum CO₂ benötigt wird. Besonders bevorzugt wird die Zusammensetzung eingesetzt, um CO₂ zur Verwendung in einer Insektenfalle herzustellen, insbesondere für eine Insektenfalle zum Anlocken blutsaugender Insekten und Arthropoden. Mit der Zusammensetzung ist es insbesondere möglich, längerfristig kontinuierlich Kohlenstoffdioxid zu erzeugen. Zudem werden kumulativ höhere Mengen an CO₂ erzeugt, als wenn die einzelnen Hefestämme in entsprechender Menge jeweils einzeln verwendet werden.

Weiterhin wird ein Verfahren zur Herstellung von CO₂ beschrieben. Hierbei wird eine wässrige Lösung aus einer Kohlenhydratquelle und einer oben beschriebenen Zusammensetzung hergestellt. Als Kohlenhydratquelle dienen beispielsweise einfache Zucker wie Fruktose und Glukose, alternativ komplexere Zucker wie Saccharose. Insbesondere kann die Verwendung von gewöhnlichem, billigen Haushaltszucker, oder aber andere Kohlenhydratquellen wie Mehl o.ä., beispielsweise Malzmehl, Getreidemehl, Maismehl oder Mehl aus anderen geeigneten Stärkequellen wie Tapioka, Yams, Maniok o.ä. vorgesehen sein.

Die nach dem Verfahren hergestellte wässrige Lösung zeichnet sich dadurch aus, dass insbesondere nach einer anfänglichen Starterphase über einen Zeitraum von mindestens 12 Stunden kontinuierlich Kohlenstoffdioxid abgegeben wird. Insbesondere werden dabei deutlich höhere Mengen an Kohlenstoffdioxid erzeugt als bei Verwendung der Einzelkomponenten a, b, c in entsprechend angepasster Dosierung. Vorzugsweise beträgt die Dauer der Starterphase bei Verwendung der erfindungsgemäßen Zusammensetzung maximal eine Stunde

Zur Herstellung von CO₂ wird eine wässrige Lösung aus einer Kohlenhydratquelle und einer oben beschriebenen Zusammensetzung erzeugt. Eine solche wässrige Lösung gibt über einen Zeitraum von mehreren Stunden, insbesondere über einen Zeitraum von mindestens 12 Stunden, kontinuierlich CO₂ ab. Besonders bevorzugt werden die Mengen an Kohlenhydratquelle und Zusammensetzung derart gewählt, dass die Mischung mindestens über 24 Stunden oder sogar mindestens über 48 Stunden kontinuierlich Kohlenstoffdioxid abgibt. Eine weitere bevorzugte Mischung aus Kohlenhydratquelle und erfindungsgemäßer Zusammensetzung in wässriger Lösung ist geeignet, eine kontinuierliche und nach Beendigung einer anfänglichen Starterphase kontinuierliche und möglichst gleichmäßige Freisetzung von Kohlenstoffdioxid über ein bis zwei Wochen zu erzielen.

Die Kohlenhydratquelle kann beispielsweise durch ein Monosaccharid, insbesondere Glucose oder Fructose gebildet werden. Besonders bevorzugt wird die Kohlenhydratquelle durch ein Disaccharid, insbesondere Saccharose, gebildet. Insbesondere kann herkömmlicher Haushaltszucker verwendet werden, wodurch eine besonders kostengünstige Herstellung von CO₂ möglich ist.

Gemäß einer Ausführungsform ist vorgesehen, dass zur Herstellung der wässrigen Lösung Wasser einer Temperatur zwischen 30 und 40 Grad Celsius verwendet wird, insbesondere Wasser einer Temperatur zwischen 33 und 38 Grad Celsius, besonders bevorzugt Wasser einer Temperatur von 35 Grad Celsius. Dies stellt eine optimale Temperatur für die Heden dar und bewirkt ein schnelles Starten der Kohlenstoffdioxid-Produktion.

Eine besonders bevorzugte Ausführungsform sieht vor, dass zur Herstellung der wässrigen Lösung 500 Gramm Zucker und 20 Gramm der Zusammensetzung in 2 Liter Wasser, welches vorzugsweise eine Temperatur von 35 Grad Celsius aufweist, vermischt werden. Vorzugsweise wird die Zusammensetzung dabei durch 10 Gramm der Komponente a); 5 Gramm der Komponente b) und 5 Gramm der Komponente c) gebildet. Besonders bevorzugt ist eine Zusammensetzung aus 10 Gramm kommerziell erhältlicher Bäckerhefe, 5 Gramm kommerziell erhältlicher Turbohefe und 5 Gramm kommerziell erhältlichem Hefeextrakt.

Mit der vorgenannten Mischung aus einer Kohlenhydratquelle und der oben beschriebenen Zusammensetzung ist es also möglich, über einen gewünschten längeren Zeitraum kontinuierlich und im Wesentlichen gleichmäßig CO₂ zu erzeugen. Dies ist insbesondere für die Verwendung von Insektenfallen für längerfristige Feldstudien vorteilhaft, bei denen Insektenfallen an einer bestimmten Stelle aufgestellt, und die in der Insektenfalle gefangenen Insekten nach einer definierten Zeit gezählt und analysiert werden sollen. Hierbei ist es wichtig, dass die Lockstoffe der Insektenfalle über den gesamten Versuchszeitraum in möglichst unveränderter Menge vorhanden sind, was bei Herstellung von Kohlenstoffdioxid unter Verwendung der im Rahmen der Anmeldung beschriebenen Zusammensetzung gewährleistet werden kann.

Die beschriebene Zusammensetzung kann vorteilhaft sowohl mit aktiven Insektenfallen als auch mit passiven Insektenfallen eingesetzt werden. Unter aktiven Insektenfallen versteht man beispielsweise mit einem Gebläse ausgestattete Insektenfallen, bei denen die angelockten Insekten in einen Sammelbeutel eingesaugt werden. Unter passiven Insektenfallen versteht man beispielsweise solche, bei denen die angelockten Insekten an einer Klebefläche o.ä. hängen bleiben oder in Fangbehältern enden, aus denen sie nicht entweichen können.

Für die Herstellung des Kohlenstoffdioxids unter Verwendung der Zusammensetzung kann die entsprechende wässrige Lösung in einem beliebigen geeigneten Behälter angesetzt werden, welcher benachbart zur Insektenfalle angeordnet werden kann und beispielsweise einen Ausgang aufweist, über welchen das Kohlenstoffdioxid gezielt an oder in die Insektenfalle einbringbar ist. Beispielsweise kann vorgesehen sein, dass ein Verbindungsschlauch an den Behälterausgang angebracht wird, so dass das Kohlenstoffdioxid an einer definierten Stelle an oder in der Insektenfalle freigesetzt wird.

Eine Ausführungsform kann die Verwendung der Zusammensetzung zur Herstellung von Kohlenstoffdioxid in speziellen Reaktionsbeuteln vorsehen. Ein solcher Reaktionsbeutel vorzugsweise aus einem dünnen Kunststoffmaterial und weiterhin eine Verschlussanordnung mit einem Deckel. Der Deckel kann vorzugsweise eine Aufnahme- oder Befestigungseinrichtung für einen Verbindungsschlauch aufweisen. Über den Verbindungsschlauch wird das in dem Reaktionsbeutel erzeugte Kohlenstoffdioxid einer Insektenfalle zugeleitet.

Beispielsweise werden solche Insektenfallen vorteilhaft im Rahmen von Studien eingesetzt, um über Nacht Flug- und/oder Schädlingsinsekten zu fangen und diese anschließend zu zählen und/oder zu kategorisieren.

Beispielsweise kann vorgesehen sein, dass das Kohlenstoffdioxid über den Verbindungsschlauch in die Insektenfalle eingeleitet und von dieser anschließend wieder abgegeben wird. Alternativ kann das freie Ende des Verbindungsschlauchs auch benachbart zu einer Insektenfalle positioniert werden, so dass das Kohlenstoffdioxid in unmittelbarer Nachbarschaft der Insektenfalle ausströmt und entsprechend dem Anlocken der Insekten dient.

Weiterhin kann vorgesehen sein, dass der Reaktionsbeutel mit einem Tragegriff ausgestattet ist, um diesen insbesondere im befüllten Zustand besser und einfacher transportieren zu können.

Eine wässrige Mischung aus einer Kohlenhydratquelle und der erfindungsgemäßen Zusammensetzung kann direkt durch Einfüllen von Wasser, Kohlenhydratquelle und Zusammensetzung in den Reaktionsbeutel hergestellt werden. In diesem Fall kann beispielsweise vorgesehen sein, dass zusätzlich ein weiterer Deckel ohne Aufnahmeöffnung zur Verfügung gestellt wird, so dass der Reaktionsbeutel im Anschluss an das Einfüllen der Bestandteile der Mischung zum Vermischen derselben kurzzeitig dicht verschlossen werden kann. Alternativ können die Komponenten auch außerhalb des Reaktionsbeutels vermischt und die Mischung in denselben eingefüllt werden.

Vorzugsweise ist vorgesehen, dass die Reaktionsbeutel mehrfach verwendet werden. Insbesondere kann dieser immer wieder neu mit einer entsprechenden Mischung aus Wasser, Kohlenhydrat und erfindungsgemäßer Zusammensetzung befüllt werden.

Alternativ ist es möglich, nach Ablauf einer definierten CO₂ Produktionszeit nur einen Teil der Mischung aus dem Reaktionsbeutel zu entnehmen und eine neue wässrige Kohlenhydratlösung zuzufügen. Die in der restlichen im Reaktionsbeutel verbleibenden Mischung enthaltenen Hefen stellen für die weitere CO₂ Produktion eine ausreichende Starterkultur dar.

Dies ist allerdings in dieser Form nicht beliebig oft möglich. Da die Menge an durch die Komponente c = den Hefeextrakt bereitgestellten Nährstoffen kontinuierlich abnimmt, kann es notwendig sein, bei mehrfacher Verwendung derselben Start-Zusammensetzung weiteren Hefeextrakt hinzuzufügen, um die optimalen Bedingungen für die CO₂ Produktion beizubehalten.

Der Reaktionsbeutel kann weiterhin mit einer Temperaturanzeige ausgestattet sein, beispielsweise mit einem Temperaturmessstreifen. Insbesondere weist dieser vorzugsweise mehrere Temperaturindikatoren auf, so dass die Temperatur an der Oberfläche des Reaktionsbeutels gemessen werden kann, wodurch entsprechende Rückschlüsse auf die innerhalb des Reaktionsbeutels vorherrschenden Temperaturen gezogen werden können. Vorzugsweise bestehen die Temperaturindikatoren aus Substanzen, die bei bestimmten Temperaturen einen reversiblen Farbwechsel ausführen, so dass die mit Temperaturanzeige ausgestatteten Reaktionsbeutel vorzugsweise mehrfach verwendet werden können.

Weiterhin kann vorgesehen sein, den Reaktionsbeutel nach dem Befüllen mit der entsprechenden Mischung aus Wasser, Kohlenhydratquelle und Zusammensetzung in einem Thermobehälter anzuordnen, um eine optimale Reaktionstemperatur zu gewährleisten und Effekt der Außentemperatur auf die CO₂ - Produktion zu verhindern.

Die Umgebung und somit die vorherrschende Außentemperatur hat einen starken Einfluss auf die CO₂ - Produktion durch die Hefen. Bei Temperaturen über 38 Grad Celsius, insbesondere bei Temperaturen über 40 Grad Celsius sterben die Hefen ab, was zu einer Verringerung der CO₂ - Produktion führt. Hierbei muss bedacht werden, dass bei der CO₂ - Produktion durch die Hefen selbst Wärme entsteht, so dass bei hohen Außentemperaturen, wie sie beispielsweise in tropischen Gebieten vorherrschen, durchaus innerhalb der Reaktionsmischung diese kritischen Temperaturen erreicht werden können.

Die Anordnung des Reaktionsbeutels in einem Thermobehälter ist aber auch vorteilhaft, wenn es über Nacht stark abkühlt, da das Wachstum und somit der Stoffwechsel der Hefen bei kalten Temperaturen, insbesondere bei Temperaturen unterhalb 30 Grad Celsius deutlich verlangsamt ist, was sich entsprechend in den Ausbeuten an Kohlenstoffdioxid widerspiegelt.

Eine weitere Ausführungsform kann vorgesehen sein, dass in die Verbindungsleitung ein Blasenzähler integriert werden kann, um die Flussrate an Kohlenstoffdioxid zu messen. Dies kann insbesondere im Rahmen von Forschungsprojekten für die genaue Analyse der Versuchsbedingungen vorteilhaft sein.

Mit der erfindungsgemäßen Zusammensetzung wird eine im Hinblick auf Kontinuität, Menge und Gleichmäßigkeit der CO₂-Abgabe optimierte CO₂-Produktion erreicht. Durch die entsprechende Mischung von mindestens zwei unterschiedlichen Hefestämmen und einer Nähstoffkomponente können die jeweils optimierten Eigenschaften der unterschiedlichen Hefestämme in sinnvoller Weise kombiniert werden. Die Zusammensetzung umfassend mindestens zwei unterschiedliche Hefestämme zeichnet sich gegenüber der Verwendung des reinen ersten Hefestammes in einer höheren CO₂ -Ausbeute aus. Gegenüber der Verwendung des reinen zweiten Hefestammes zeichnet sich die Zusammensetzung insbesondere dadurch aus, dass die Produktion von CO₂ deutlich schneller startet.

Durch entsprechendes Anpassen der Verhältnisse von Zusammensetzung zu Kohlenhydratquelle und/oder durch Anpassen der Verhältnisse der einzelnen Komponenten a, b und c innerhalb der Zusammensetzung kann insbesondere eine kontinuierliche und im Wesentlichen gleichmäßige CO₂ - Produktion über unterschiedliche gewünschte Zeiträume, insbesondere über Zeiträume von mehreren Tagen bis hin zu ein bis zwei Wochen erreicht werden.

Die Zusammensetzung ist insbesondere kostengünstig herstellbar, da beispielsweise geringere Mengen der in der Regel teureren Komponente b als von der in der Regel billiger zu beziehenden Komponente in der Mischung enthalten sind. Mit der Zusammensetzung ist das CO₂ deutlich kostengünstiger herstellbar als bei Verwendung entsprechender Mengen an Trockeneis oder im Gaszylinder komprimiertem CO₂.

Ein weiterer Vorteil ist die einfache Entsorgung der Mischung. Da es sich hierbei um eine Mischung aus Wasser, Alkohol und Hefen handelt, kann dieses einfach entsorgt werden, ohne dass hierbei spezielle Vorschriften beachtet werden müssen.

Es sei an dieser Stelle ausdrücklich erwähnt, dass alle Aspekte und Ausführungsvarianten, die im Zusammenhang mit der erfindungsgemäßen Vorrichtung erläutert wurden, gleichermaßen Teilaspekte des erfindungsgemäßen Verfahrens betreffen oder sein können. Wenn daher an einer Stelle bei der Beschreibung oder auch bei den Anspruchsdefinitionen zur erfindungsgemäßen Vorrichtung von bestimmten Aspekten und/oder Zusammenhängen und/oder Wirkungen die Rede ist, so gilt dies gleichermaßen für das erfindungsgemäße Verfahren.

In umgekehrter Weise gilt dasselbe, so dass auch alle Aspekte und Ausführungsvarianten, die im Zusammenhang mit dem erfindungsgemäßen Verfahren erläutert wurden, gleichermaßen Teilaspekte der erfindungsgemäßen Vorrichtung betreffen oder sein können. Wenn daher an einer Stelle bei der Beschreibung oder auch bei den Anspruchsdefinitionen zum erfindungsgemäßen Verfahren von bestimmten Aspekten und/oder Zusammenhängen und/oder Wirkungen die Rede ist, so gilt dies gleichermaßen für die erfindungsgemäße Vorrichtung.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Figur 1 zeigt graphisch die vermittels einer erfinderischen Zusammensetzung hergestellten Mengen an Kohlenstoffdioxid.
Figur 2 zeigt einen Reaktionsbeutel zur Herstellung von Kohlenstoffdioxid.
Figur 3 zeigt die Verwendung der Zusammensetzung in einer Insektenfalle.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die erfindungsgemäße Vorrichtung oder das erfindungsgemäße Verfahren ausgestaltet sein können und stellen keine abschließende Begrenzung dar.

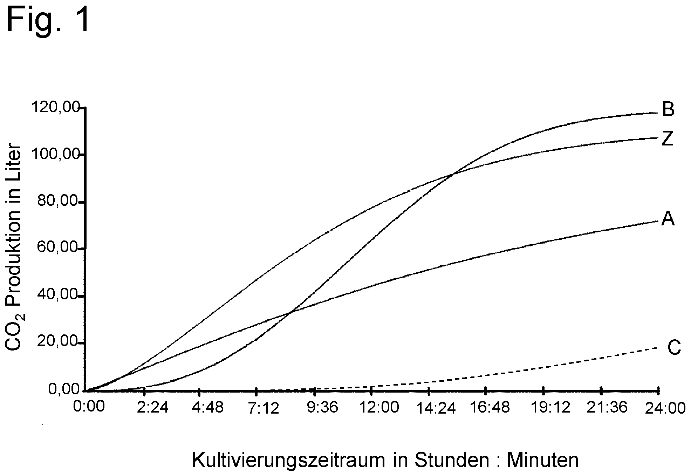

Figur 1 zeigt graphisch die vermittels einer erfinderischen Zusammensetzung hergestellten Mengen an Kohlenstoffdioxid. In dem Graph stellt die X-Achse den Kultivierungszeitraum in Stunden und Minuten dar. Die Y-Achse stellt die CO₂ Produktion in Liter dar. Es wurde jeweils eine wässrige Kohlenhydratlösung hergestellt, die jeweils mit einer definierten Menge einer Einzelkomponente a), b) oder c) der Zusammensetzung oder mit einer definierten Menge der Zusammensetzung gemischt wurde. Anschließend wurde die CO₂ Produktion in Liter über einen Kultivierungszeitraum von 24 Stunden gemessen und die Messwerte graphisch dargestellt.

Kurve A zeigt die CO₂ Produktion bei Verwendung einer definierten Menge X einer Instant-Backhefe. Kurve B zeigt die CO₂ Produktion bei Verwendung einer definierten Menge X einer sogenannten Turbo-Hefe. Kurve C zeigt die CO₂ Produktion bei Verwendung einer definierten Menge X eines Hefeextraktes und Kurve Z zeigt die CO₂ Produktion bei Verwendung einer definierten Menge X einer Zusammensetzung umfassend 0,5X Instant-Backhefe; 0,25X Turbo-Hefe und 0,25X Hefeextrakt.

Kurve A zeigt deutlich, dass die Bäckerhefe insbesondere darauf optimiert ist, unter Luftabschluss und in kurzer Zeit große Mengen an Kohlenstoffdioxid zu erzeugen. Insbesondere startet die CO₂ Produktion bei der Bäckerhefe im Wesentlichen sofort nach dem Zumischen der Bäckerhefe zu der Kohlenhydratlösung und steigt über den Zeitraum von 24 Stunden im Wesentlichen kontinuierlich und gleichmäßig an.

Unter Turbohefe versteht man eine Mischung aus einer Hefe und einer Hefenahrung, die für die Produktion von Alkohol optimiert ist und einen Alkoholgehalt von mindestens 14% tolerieren. Beispielsweise besteht eine solche Turbohefe zu 58% aus einer Brennhefe, 20% Carbamid, 16% Phosphaten, 3% Sulfaten, 2% Karbonaten, sowie 1% Vitaminen und Spurenelementen

In Kurve B lässt sich erkennen, dass die Produktion von Kohlenstoffdioxid erst verzögert startet, insbesondere geht die CO₂ Produktion erst nach circa acht Stunden in den linearen Bereich über und nach weiteren acht Stunden tritt bereits die Sättigung ein. Die Sättigung ergibt sich dabei insbesondere durch den Verbrauch der Kohlenhydratquelle.

Weiterhin ist in Kurve C die CO₂ Produktion bei Verwendung eines Hefeextraktes als Komponente c) dargestellt. Dabei handelt es sich um totes Hefezellmaterial, was je nach Hersteller mehr oder weniger stark aufgeschlossen ist, aber in der Regel keine aktiven Hefezellen enthält. Die nach circa acht Stunden beginnende geringfügige CO₂ Produktion erklärt sich dadurch, dass bei der Herstellung der Kohlenhydratlösung und Zugabe des Hefeextraktes nicht steril gearbeitet wurde. Somit gelangen Organismen aus der Umgebung, beispielsweise Luft, in die durch den Hefeextrakt gebildete Nährlösung. Die Organismen vermehren sich in der Nährlösung und produzieren durch die normale Zellatmung ebenfalls CO₂. Es wurde nicht unter sterilen Bedingungen gearbeitet, da bei der Verwendung der Zusammensetzung mit Insektenfallen in der Regel auch nicht steril gearbeitet werden wird oder kann.

Der Vergleich mit der Kurve Z, der die CO₂ Produktion bei Verwendung einer Zusammensetzung aus 0,5 Teilen Instant-Backhefe; 0,25 Teilen Turbo-Hefe und 0,25 Teilen Hefeextrakt lässt erkennen, dass die CO₂ Produktion mit der Zusammensetzung sofort startet und nach circa einer Stunde bereits in den Linearbereich eintritt. Mit der Zusammensetzung können insbesondere in den ersten zwölf Stunden kontinuierlich und im Wesentlichen gleichmäßig deutlich höhere Mengen an CO₂ erzeugt werden, als bei alleiniger Verwendung von Bäckerhefe (Kurve A). Weiterhin ist die CO₂ Produktion in den ersten zwölf Stunden auch gegenüber der alleinigen Verwendung von Turbohefe (Kurve B) optimiert, da hierbei die CO₂- Produktion erst verzögert startet.

Beispielsweise werden 500g Haushaltszucker in zwei Liter warmem Wasser, insbesondere in Wasser mit einer Temperatur von ungefähr 35 Grad Celsius, gelöst und jeweils 20 Gramm der Einzelkomponenten oder 20 Gramm der Zusammensetzung zugefügt. Mit einer solchen Mischung können über einen Zeitraum von 24 Stunden circa 200g CO₂ erzeugt werden.

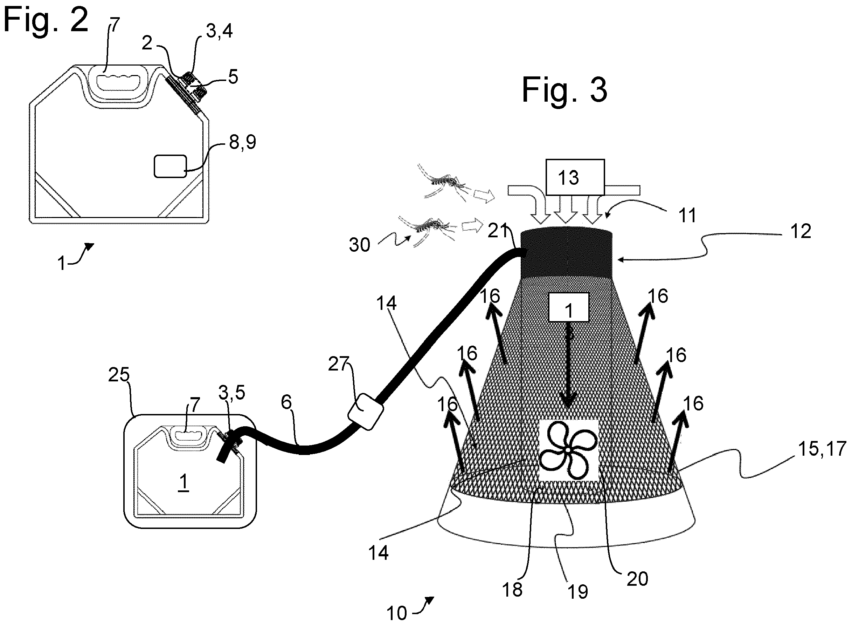

Figur 2 zeigt einen Reaktionsbeutel 1 zur Herstellung von Kohlenstoffdioxid vermittels der vorbeschriebenen Zusammensetzung. Der Reaktionsbeutel 1 besteht vorzugsweise aus einem dünnen Kunststoffmaterial. Der Reaktionsbeutel 1 weist weiterhin eine Verschlussanordnung 2 mit einem Deckel 3 auf. Vorzugsweise handelt es sich hierbei um einen Schraubdeckel 4, der eine Aufnahmeöffnung 5 für einen Verbindungsschlauch 6 (vgl. Figur 3) umfasst.

Weiterhin kann vorgesehen sein, dass der Reaktionsbeutel 1 mit einem Tragegriff 7 ausgestattet ist, um den Reaktionsbeutel 1 insbesondere im befüllten Zustand besser und einfacher transportieren zu können.

Die wässrige Mischung aus einer Kohlenhydratquelle, insbesondere Zucker, und erfindungsgemäßer Zusammensetzung kann direkt in diesem hergestellt werden, indem Wasser, Zucker und Zusammensetzung in den Reaktionsbeutel 1 eingefüllt und in diesem miteinander vermischt werden. In diesem Fall kann vorgesehen sein, dass zusätzlich ein weiterer Deckel ohne Aufnahmeöffnung zur Verfügung gestellt wird, so dass der Reaktionsbeutel 1 im Anschluss an das Einfüllen der Bestandteile der Mischung zum Vermischen derselben kurzzeitig dicht verschlossen werden kann. Alternativ können die Bestandteile auch außerhalb des Reaktionsbeutels 1 vermischt und die Mischung anschließend in den Reaktionsbeutel 1 eingefüllt werden.

Vorzugsweise ist vorgesehen, dass der Reaktionsbeutel 1 mehrfach verwendet werden kann. Insbesondere kann der Reaktionsbeutel 1 immer wieder neu mit einer entsprechenden Mischung aus Wasser, Kohlenhydratquelle und Zusammensetzung befüllt werden.

Alternativ ist es möglich, nach Ablauf einer definierten CO₂ Produktionszeit nur einen Teil der Mischung aus dem Reaktionsbeutel 1 zu entnehmen und eine neue wässrige Kohlenhydratlösung zuzufügen. Die in der restlichen im Reaktionsbeutel 1 verbleibenden Mischung enthaltenen Hefen stellen für die weitere CO₂ Produktion eine ausreichende Starterkultur dar. Gegebenenfalls ist dies allerdings in dieser Form nicht beliebig oft möglich. Da die Menge an Nährstoffen, die durch die Komponente c) bereitgestellt werden, auf diese Weise kontinuierlich abnimmt. Bei mehrfacher Verwendung derselben Start-Zusammensetzung kann es deshalb notwendig sein, weiteren Hefeextrakt hinzuzufügen, um die optimalen Bedingungen für die CO₂ Produktion beizubehalten.

Der Reaktionsbeutel 1 kann weiterhin mit einer Temperaturanzeige 8 ausgestattet sein, beispielsweise mit einem Temperaturmessstreifen 9. Insbesondere weist dieser Temperaturmessstreifen 9 vorzugsweise mehrere Temperaturindikatoren auf, so dass die Temperatur an der Oberfläche des Reaktionsbeutels 1 gemessen werden kann, wodurch entsprechende Rückschlüsse auf die innerhalb des Reaktionsbeutels 1 vorherrschenden Temperaturen gezogen werden können. Vorzugsweise bestehen die Temperaturindikatoren aus Substanzen, die bei bestimmten Temperaturen einen reversiblen Farbwechsel ausführen, so dass die mit Temperaturanzeige 8 ausgestatteten Reaktionsbeutel 1 vorzugsweise mehrfach verwendet werden können.

Figur 3 zeigt die Verwendung der erfindungsgemäßen Zusammensetzung zur Herstellung von Kohlenstoffdioxid für eine Insektenfalle 10. Im dargestellten Ausführungsbeispiel handelt es sich insbesondere um eine Insektenfalle 10 zum Anlocken und/oder Fangen von Flug- und/oder Schädlingsinsekten 30 wie etwa von Stechmücken, Moskitos, Gelbfiebermücken, Zika-Mücken, Tigermücken oder andere blutsaugende Insekten, wie beispielsweise blutsaugende Wanzen, blutsaugende Fliegen, Flöhe, Läuse, Gnitzen, Sandmücken etc. etc.

Die Insektenfalle 10 weist eine obere kreisrunde Ansaugöffnung 11 auf, die sich in einen vertikal nach unten führenden zylindrischen Ansaugkanal 12 fortsetzt, in dem eine die Ansaugöffnung 11 mit Unterdruck bzw. mit einer Saugströmung 13 beaufschlagende Luftströmung herrscht, die zu einem Innenraum 14 der Insektenfalle 10 bzw. dort hinein führt. Außerdem ist die Insektenfalle 10 mit einer kegelstumpfförmigen Außenwandung 15 ausgestattet, die eine für ausströmende Luft 16 zumindest teilweise durchlässige Oberfläche aufweist, insbesondere gebildet durch eine netzartige Struktur 17, deren Maschenweite groß genug ist, dass eine ausreichende ausströmende Luftströmung 16 dort hindurchpassieren kann, deren Maschenweite jedoch zuverlässig ein Entweichen der im Innenraum 14 der Falle 10 gefangenen Insekten 30 verhindert.

Die Außenwandung 15 umschließt den Ansaugkanal 12 in Nähe der Ansaugöffnung 11 und umhüllt den Ansaugkanal 12 im sich nach unten fortsetzenden weiteren Bereich in sich änderndem radialen Abstand, so dass sich die Außenwandung 15 nach unten hin kegelförmig aufweitet. Außerdem ist die Insektenfalle 10 mit einer sich an die Außenwand 15 anschließenden und weitgehend für ein- oder ausströmende Luft undurchlässigen und der Ansaugöffnung 11 gegenüberliegenden Bodenseite 18 ausgestattet, die beabstandet ist von einer in den Innenraum 14 der Insektenfalle 10 reichenden offenen unteren Stirnseite 19 des Ansaugkanals 12. Die Bodenseite 18 ist im gezeigten Ausführungsbeispiel der Insektenfalle 10 eben und zylindrisch geformt, so dass sie senkrecht zur Längsachse des Ansaugkanals 12 ausgerichtet ist.

Innerhalb des Ansaugkanals 12 kann mindestens ein die Saugströmung 13 erzeugendes Gebläse 20 angeordnet sein. Die Saugströmung 13 weist eine Luftgeschwindigkeit auf, die es den angelockten Insekten 30 nach Möglichkeit deutlich erschwert, in der Nähe der Ansaugöffnung 11 zu entfliehen. Vielmehr sollen sie mit Hilfe der ausreichend starken Saugströmung 13 in den Innenraum 14 der Falle 10 eingesaugt und dort am Zurückfliegen aus dem Innenraum 14 zuverlässig gehindert werden. Innerhalb der Falle 10 können geeignete Mittel zum Festhalten oder Abtöten der gefangenen Insekten 30 angeordnet sein, die hier jedoch nicht dargestellt sind.

Die Insektenfalle 10 kann hängend oder stehend montierbar sein, so dass die Ansaugöffnung 11 nach oben gerichtet ist und der Ansaugkanal 12 vorzugsweise in etwa vertikaler Richtung verläuft, und wobei die Bodenseite 18 einen unteren horizontalen Abschluss der Falle 10 bildet.

Die ausströmende Luft 16 stellt bereits einen Lockreiz für die Insekten dar. Als zusätzlicher Lockreiz wird die ausströmende Luft zusätzlich mit Kohlenstoffdioxid angereichert, indem über eine entsprechende Zuführung Kohlenstoffdioxid in die Insektenfalle 10 eingeleitet wird. Dieses vermischt sich innerhalb der Insektenfalle 10 mit der durch die Saugströmung 13 angesaugten Luft und strömt über die Außenwandung 15 aus der Insektenfalle aus.

Beispielsweise wird ein Reaktionsbeutel 1 mit einer wässrigen Lösung umfassend einen Zucker als Kohlenhydratquelle und die im Rahmen der Anmeldung beschriebene erfindungsgemäße Zusammensetzung befüllt. Über die Aufnahmeöffnung 5 wird ein freies Ende eines Verbindungsschlauchs 6 in den Reaktionsbeutel 1 eingeführt und am Reaktionsbeutel 1 befestigt. Das andere freie Ende des Verbindungsschlauchs 6 wird an einer entsprechenden Aufnahmeöffnung 21 der Insektenfalle 10 befestigt.

Das innerhalb des Reaktionsbeutels 1 entstehende Kohlenstoffdioxid wird durch den Verbindungsschlauch 6 in die Insektenfalle 10 eingeleitet und mit der ausströmenden Luft 16 über die Au0enwandung 15 wieder aus der Insektenfalle 10 ausgeleitet, wodurch es seine anlockende Wirkung auf die Flug- und/oder Schädlingsinsekten 30 ausüben kann.

Beispielsweise werden solche Insektenfallen 10 vorteilhaft im Rahmen von Studien eingesetzt, um über Nacht Flug- und/oder Schädlingsinsekten 30 zu fangen. Die Flug- und/oder Schädlingsinsekten 30 werden anschließend im Rahmen der Studie gezählt und kategorisiert. Die gleichmäßige Produktion von Kohlenstoffdioxid startet nach ungefähr einer Stunde. Mit den Vorbereitungen der Insektenfalle 10 sollte somit idealerweise circa eine Stunde vor Beginn der Zählung begonnen werden.

Der Reaktionsbeutel 1 kann zusätzlich in einem Thermobehälter 25 angeordnet werden. In dem Thermobehälter 25 kann eine optimale Reaktionstemperatur gewährleistet werden. Insbesondere wird dadurch ein Effekt der Außentemperatur auf die CO₂ - Produktion verhindert.

Die Umgebung und somit die vorherrschende Außentemperatur hat einen starken Einfluss auf die CO₂ - Produktion durch die Hefen. Bei Temperaturen über 38 Grad Celsius, insbesondere bei Temperaturen über 40 Grad Celsius sterben die Hefen ab, was zu einer Verringerung der CO₂ - Produktion führt. Hierbei muss bedacht werden, dass bei der CO₂ - Produktion durch die Hefen selbst Wärme entsteht, so dass bei hohen Außentemperaturen, wie sie beispielsweise in tropischen Gebieten vorherrschen, durchaus innerhalb der Reaktionsmischung diese kritischen Temperaturen auch nachts erreicht werden können.

Die Anordnung des Reaktionsbeutels 1 in einem Thermobehälter 25 ist auch vorteilhaft, wenn es beispielsweise über Nacht stark abkühlt, da das Wachstum und somit der Stoffwechsel der Hefen bei kalten Temperaturen, insbesondere bei Temperaturen unterhalb 30 Grad Celsius, deutlich verlangsamt ist, was sich entsprechend in einer Verringerung der CO₂ - Produktion wiederspiegelt.

Weiterhin kann vorgesehen sein, dass in die Verbindungsleitung 6 ein Blasenzähler 27 integriert werden kann, um die Flussrate an Kohlenstoffdioxid zu messen.

Mit der erfindungsgemäßen Zusammensetzung wird eine im Hinblick auf Kontinuität, Menge und Gleichmäßigkeit der CO₂ -Abgabe optimierte CO₂ - Produktion erreicht. Durch die entsprechende Kombination von mindestens zwei unterschiedlichen Hefestämmen und einer Nähstoffkomponente in der erfindungsgemäßen Zusammensetzung können die jeweils optimierten Eigenschaften der unterschiedlichen Hefestämme in sinnvoller Weise kombiniert werden. Die Zusammensetzung zeichnet sich gegenüber der Verwendung von reiner Bäckerhefe in einer höheren CO₂ -Ausbeute aus. Gegenüber der Verwendung von reiner Turbohefe zeichnet sich die Zusammensetzung insbesondere dadurch aus, dass die Produktion von CO₂ deutlich schneller startet und insbesondere deutlich schneller in den linearen, gleichmäßigen Produktionsbereich übergeht.

Durch entsprechendes Anpassen der Mengenverhältnisse von der Zusammensetzung in Bezug zur Kohlenhydratquelle und/oder durch Anpassen der Verhältnisse der einzelnen Komponenten a), b) und c) innerhalb der Zusammensetzung kann insbesondere eine kontinuierliche und im Wesentlichen gleichmäßige CO₂ - Produktion über unterschiedliche gewünschte Zeiträume, insbesondere über Zeiträume von mehreren Tagen bis hin zu ein bis zwei Wochen erreicht werden.

Die Zusammensetzung ist kostengünstig herstellbar, da in der Zusammensetzung beispielsweise geringere Mengen von Turbohefe im Vergleich zur billiger zu beziehenden Bäckerhefe enthalten sind. Mit der Zusammensetzung ist das CO₂ deutlich kostengünstiger herstellbar als bei Verwendung entsprechender Mengen an Trockeneis oder im Gaszylinder komprimiertem CO₂.

Ein weiterer Vorteil ist die einfache Entsorgung der Mischung. Da es sich hierbei um eine Mischung aus Wasser, Alkohol und Hefen handelt, kann dieses einfach entsorgt werden, ohne dass hierbei spezielle Vorschriften beachtet werden müssen.

Die Ausführungsformen, Beispiele und Varianten der vorhergehenden Absätze, die Ansprüche oder die folgende Beschreibung und die Figuren, einschließlich ihrer verschiedenen Ansichten oder jeweiligen individuellen Merkmale, können unabhängig voneinander oder in beliebiger Kombination verwendet werden. Merkmale, die in Verbindung mit einer Ausführungsform beschrieben werden, sind für alle Ausführungsformen anwendbar, sofern die Merkmale nicht unvereinbar sind.

Wenn auch im Zusammenhang der Figuren generell von "schematischen" Darstellungen und Ansichten die Rede ist, so ist damit keineswegs gemeint, dass die Figurendarstellungen und deren Beschreibung hinsichtlich der Offenbarung der Erfindung von untergeordneter Bedeutung sein sollen. Der Fachmann ist durchaus in der Lage, aus den schematisch und abstrakt gezeichneten Darstellungen genug an Informationen zu entnehmen, die ihm das Verständnis der Erfindung erleichtern, ohne dass er etwa aus den gezeichneten und möglicherweise nicht exakt maßstabsgerechten Größenverhältnissen der Vorrichtung und/oder Teilen der Vorrichtung oder anderer gezeichneter Elemente in irgendeiner Weise in seinem Verständnis beeinträchtigt wäre. Die Figuren ermöglichen es dem Fachmann als Leser somit, anhand der konkreter erläuterten Umsetzungen des erfindungsgemäßen Verfahrens und der konkreter erläuterten Funktionsweise der erfindungsgemäßen Vorrichtung ein besseres Verständnis für den in den Ansprüchen sowie im allgemeinen Teil der Beschreibung allgemeiner und/oder abstrakter formulierten Erfindungsgedanken abzuleiten.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichen

- 1: Reaktionsbeutel
- 2: Verschlussanordnung
- 3: Deckel
- 4: Schraubdeckel
- 5: Aufnahmeöffnung
- 6: Verbindungsschlauch
- 7: Tragegriff
- 8: Temperaturanzeige
- 9: Temperaturmessstreifen
- 10: Insektenfalle
- 11: Ansaugöffnung
- 12: Ansaugkanal
- 13: Saugströmung
- 14: Innenraum
- 15: Außenwandung
- 16: ausströmende Luft
- 17: netzartige Struktur
- 18: Bodenseite
- 19: Stirnseite
- 20: Gebläse
- 21: Aufnahmeöffnung
- 25: Thermobehälter
- 27: Blasenzähler
- 30: Flug- und/oder Schädlingsinsekten
- A: CO₂ Produktion bei Verwendung von Instant-Backhefe
- B: CO₂ Produktion bei Verwendung von Turbo-Hefe
- C: CO₂ Produktion bei Verwendung von Hefeextrakt
- Z: CO₂ Produktion bei Verwendung der erfindungsgemäßen Zusammensetzung

## Patentansprüche

1. Zusammensetzung zur Herstellung von CO₂, umfassend:
• eine Komponente a), die Komponente a) umfassend mindestens ersten Hefestamm, wobei der mindestens eine erste Hefestamm eine geringe Toleranz von weniger als 100g Alkohol pro Liter aufweist,
• eine Komponente b), die Komponente b) umfassend mindestens einen zweiten Hefestamm, wobei der mindestens eine zweite Hefestamm eine hohe Toleranz von mehr als 100g Alkohol pro Liter aufweist,
• eine Komponente c), die Komponente c) umfassend mindestens eine Nährstoffquelle.

2. Zusammensetzung nach Anspruch 1, wobei Komponente a) durch eine Bäckerhefe gebildet ist.

3. Zusammensetzung nach Anspruch 1 oder 2, wobei Komponente b) eine Brennhefe, eine Bierhefe und/oder eine Weinhefe umfasst.

4. Zusammensetzung nach einem der voranstehenden Ansprüche, wobei Komponente b) durch eine Turbohefe gebildet ist.

5. Zusammensetzung nach einem der voranstehenden Ansprüche, wobei Komponente c) durch eine Turbohefe gebildet ist.

6. Zusammensetzung nach einem der voranstehenden Ansprüche, wobei Komponente c) durch einen Hefeextrakt gebildet ist.

7. Zusammensetzung nach einem der voranstehenden Ansprüche, wobei die Komponenten a : b : c jeweils in gefriergetrockneter Form vorliegen.

8. Zusammensetzung nach einem der voranstehenden Ansprüche, wobei die Komponenten a : b : c in einem Mengenverhältnis oder Gewichtsverhältnis von 1-10: 1-10: 1-10 in der Zusammensetzung vorhanden sind.

9. Zusammensetzung nach einem der Ansprüche 1 bis 7, wobei die Komponenten a : b : c in einem Mengenverhältnis von 2: 1: 1 oder 1: 2: 1 oder 1: 1: 2 in der Zusammensetzung vorhanden sind.

10. Zusammensetzung nach einem der voranstehenden Ansprüche, wobei der der erste Hefestamm und/oder der zweite Hefestamm osmotolerant sind und relativ hohe Anfangskonzentrationen an Zucker vertragen.

11. Verwendung einer Zusammensetzung zur Herstellung von CO₂ für eine Insektenfalle, insbesondere für eine Insektenfalle zum Anlocken blutsaugender Insekten und Arthropoden, die Zusammensetzung umfassend
• eine Komponente a), die Komponente a) umfassend mindestens ersten Hefestamm, wobei der mindestens eine erste Hefestamm eine geringe Toleranz von weniger als 100g Alkohol pro Liter aufweist,
• eine Komponente b), die Komponente b) umfassend mindestens einen zweiten Hefestamm, wobei der mindestens eine zweite Hefestamm eine hohe Toleranz von mehr als 100g Alkohol pro Liter aufweist,
• eine Komponente c), die Komponente c) umfassend mindestens eine Nährstoffquelle.

12. Verfahren zur Herstellung von CO₂, wobei eine wässrige Lösung aus
• einer Kohlenhydratquelle und
• einer Zusammensetzung umfassend
eine Komponente a), die Komponente a) umfassend mindestens ersten Hefestamm, wobei der mindestens eine erste eine geringe Toleranz von weniger als 100g Alkohol pro Liter aufweist,
eine Komponente b), die Komponente a) umfassend mindestens einen zweiten Hefestamm, der eine hohe Toleranz von mehr als 100g Alkohol pro Liter aufweist,
eine Komponente c), die Komponente a) umfassend mindestens eine Kohlenhydratquelle
hergestellt wird.

13. Verfahren nach Anspruch 11, wobei die Kohlenhydratquelle durch ein Monosaccharid, insbesondere Glucose oder Fructose, oder durch ein Disaccharid, insbesondere Saccharose, durch ein Mehl oder eine andere geeignete Stärkequelle gebildet wird.

14. Verfahren nach Anspruch 11 oder 12, wobei zur Herstellung der wässrigen Lösung Wasser einer Temperatur zwischen 30 und 40 Grad Celsius verwendet wird, insbesondere Wasser einer Temperatur zwischen 33 und 38 Grad Celsius, besonders bevorzugt Wasser einer Temperatur von 35 Grad Celsius.

15. Verfahren nach einem der Ansprüche 11 bis 13, wobei zur Herstellung der wässrigen Lösung 500 Gramm Zucker und 20 Gramm der Zusammensetzung in 2 Liter Wasser vermischt werden.
